# FASCICULE DE BREVET EUROPEEN

(11) **EP 1 908 402 B1**
(45) Date de publication et mention de la délivrance du brevet: **09.12.2015**
(21) Numéro de dépôt: 06121871.5
(22) Date de dépôt: 06.10.2006
(51) Int. Cl.: A61B 5/00, A61B 5/024, A61B 5/11

(54) **Méthode et dispositif intégré de mesure d'une pulsation cardiaque**
Verfahren und Vorrichtung zur Ausmessung des Herzschlags
Method and device for measuring the heartbeat

(43) Date de publication de la demande: 09.04.2008
(73) Titulaire: ETA SA Manufacture Horlogère Suisse, 2540 Grenchen (CH)
(72) Inventeur: Renevey, Philippe, 1005, Lausanne (CH); Vetter, Rolf, 1116, Cottens (CH); Galli, Reto, 3012, Bern (CH)
(74) Mandataire: Ravenel, Thierry Gérard Louis

(56) Documents cités:
- EP-A1- 0 659 384
- EP-A1- 0 922 433
- EP-A1- 0 941 694
- US-A- 4 338 950
- US-A- 5 807 267
- US-A1- 2006 084 879

## Description

La présente invention se rapporte au domaine du traitement du signal. Elle concerne plus particulièrement une méthode et un dispositif intégré de mesure d'une pulsation cardiaque durant la pratique d'une activité sportive.

Les dispositifs de mesure du rythme cardiaque comportent généralement une sonde externe, fixée, par exemple, à la poitrine ou à l'oreille, et connectée à une unité de traitement du signal et d'affichage. Ces dispositifs sont relativement encombrants et incommodes.

Récemment, des dispositifs intégrés ont fait leur apparition. Par dispositif intégré, on entend un dispositif formé d'un unique module effectuant, au même point, la mesure d'un signal, son traitement et l'affichage du résultat. De tels dispositifs sont, par exemple, portés au poignet à la façon d'une montre. Leur fonctionnement est basé sur une technique optique de mesure d'une donnée physiologique appelée photoplethysmographie (ou PPG). Le principe en est le suivant : Une source optique émet un rayonnement infrarouge se propageant à l'intérieur des tissus humains dans lesquels il subit de la diffusion et de l'absorption. L'interaction entre le rayonnement et les tissus dépend du type et du volume de tissu traversé. Un détecteur optique mesure le signal en sortie des tissus. Ce signal contient une information sur les tissus traversés. En l'absence de mouvement du porteur, il comporte une composante continue, issue des tissus statiques, et une composante périodique, issue des tissus pulsatiles, typiquement le sang. Cette dernière composante permet la mesure du rythme cardiaque.

Un inconvénient lié à la méthode de mesure du rythme cardiaque par PPG est sa sensibilité aux mouvements du sujet sur lequel la mesure est pratiquée. Ces mouvements génèrent des artefacts dont la contribution au signal excède souvent la contribution utile de la pulsation cardiaque, d'un ordre de grandeur. Le signal optique doit ainsi être traité de manière à éliminer la contribution due aux mouvements. On parle de rehaussement du signal optique. A cet effet, les dispositifs intègrent généralement un accéléromètre à trois dimensions apte à délivrer un signal représentatif du mouvement effectué au point où le signal optique est mesuré.

Différentes méthodes de rehaussement du signal optique à l'aide du signal d'accélération ont été divulguées à ce jour. On citera, par exemple, le document US 7 018 338 qui décrit une méthode basée sur la modélisation non linéaire du signal d'accélération. Une autre méthode, décrite dans le document WO 2006/044677, transpose les signaux optique et d'accélération, du domaine temporel fréquentiel par transformation de Fourier (FFT), en vue d'éliminer les artefacts liés au mouvement. Le document EP0922433 décrit par ailleurs un dispositif pour la mesure de pulsations cardiaques durant la pratique d'une activité sportive. Un triple filtrage est effectué séquentiellement pour respectivement éliminer le bruit généré par des variations de fréquence, effectuer un filtrage sur une pseudo-plage de fréquence suivant le rythme cardiaque mesuré, et enfin une étape d'élimination de la composante du signal associé au mouvement du corps. Le document US4338950 décrit un autre appareil de mesure de pulsation cardiaque comprenant deux capteurs, le premier détectant un signal électrique composite mélangeant les battements du coeur et le mouvement du corps de l'utilisateur, et un deuxième capteur détectant les mouvements périodiques du corps de l'utilisateur. La mesure des battements cardiaques est obtenue grâce à la suppression du signal lié au mouvement du corps par un filtre qui soustrait des portions correspondantes du signal composite séparées par des intervalles de temps multiples de la période du deuxième signal, déterminée au préalable.

De telles méthodes de rehaussement du signal optique sont complexes et nécessitent une puissance de calcul considérable. Par conséquent, la mise en oeuvre de la méthode de mesure du rythme cardiaque par PPG s'avère extrêmement difficile. Des améliorations sont donc nécessaires en vue de la simplifier et l'optimiser. La présente invention se base sur une observation originale pour proposer une méthode de mesure d'un rythme cardiaque par PPG répondant à cette exigence. En effet, on constate que les méthodes actuelles ne prennent pas en compte l'activité sportive pratiquée. Or, le mouvement effectué durant une telle activité, d'une part varie considérablement en fonction de l'activité pratiquée et d'autre part, possède ses spécificités. De ce fait, la connaissance de l'activité pratiquée recèle des informations non prises en considération dans les méthodes divulguées. Ces informations peuvent s'avérer intéressantes, en vue de simplifier ou adapter les méthodes de mesure du rythme cardiaque par PPG, et en particulier, les méthodes de rehaussement du signal optique. La présente invention propose donc une méthode de mesure du rythme cardiaque par PPG dont les performances sont améliorées grâce à la prise en compte du type d'activité sportive pratiquée, en fonction d'une sélection par l'utilisateur parmi un choix d'activités présélectionnées.

Plus précisément, l'invention concerne une méthode de mesure d'une pulsation cardiaque durant une activité sportive selon la revendication 1 dont le calcul de la pulsation cardiaque est basé sur un algorithme comprenant un bloc de traitement pour séparer des composantes relatives, respectivement, à la pulsation cardiaque et au mouvement effectué, appelé bloc de rehaussement du signal optique comprenant:
- une étape de calcul de la fréquence fondamentale du mouvement,
- une étape de calcul des harmoniques, et
- une étape d'élimination des fréquences du mouvement ainsi calculées par filtrage dudit signal optique à l'aide d'un filtre de type peigne, comprenant des bandes passantes et des bandes de réjection, dont la largeur est adaptée au type d'activité pratiquée.

Grâce à ces caractéristiques, la méthode selon l'invention peut gagner en rapidité, en fiabilité ou en précision de la mesure du rythme cardiaque.

L'invention concerne également un dispositif intégré pour la mise en oeuvre d'une telle méthode, comportant :
- une source lumineuse,
- un photo-détecteur fournissant un signal optique comprenant une composante relative à ladite pulsation cardiaque et une composante relative au mouvement effectué durant ladite activité sportive,
- un accéléromètre fournissant un signal d'accélération contenant une indication relative au mouvement effectué durant ladite activité sportive, et
- un processeur apte à traiter lesdits signaux de manière à calculer ladite pulsation cardiaque à partir dudit signal optique et à l'aide du signal d'accélération, par séparation des composantes relatives, respectivement, à la pulsation cardiaque et au mouvement effectué,
- des moyens de détermination de ladite activité sportive comportant un sélecteur manuel de l'activité sportive pratiquée formé par un organe de commande externe, et qui sont agencés pour fournir au processeur une indication du type d'activité pratiquée pour adapter le calcul de la pulsation cardiaque en fonction de l'indication du type d'activité.

D'autres caractéristiques et avantages de la présente invention ressortiront plus clairement de la description détaillée qui suit d'un exemple de réalisation d'un dispositif de mesure d'un rythme cardiaque selon l'invention, cet exemple étant donné à titre purement illustratif et non limitatif seulement, en liaison avec le dessin annexé sur lequel :
- les figures 1 et 2 sont des vues respectivement de dessous et en coupe du dispositif selon l'invention,
- la figure 3 est un diagramme illustrant un algorithme de calcul du rythme cardiaque utilisé par le dispositif selon l'invention, et
- les figures 4a, 4b et 4c se rapportent à une étape de l'algorithme représenté en figure 3.

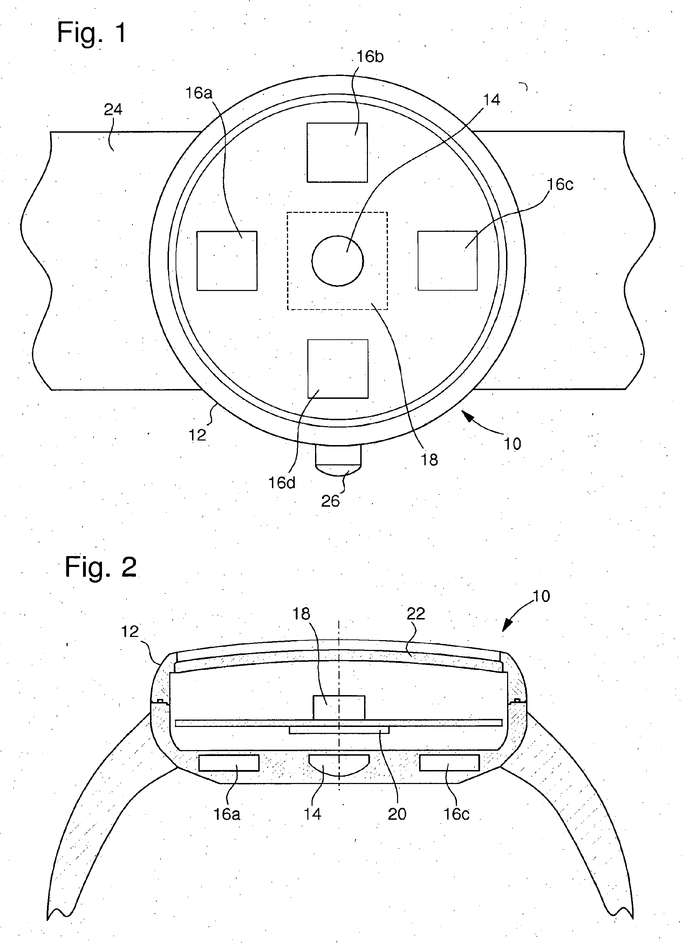

Le dispositif intégré de mesure d'un rythme cardiaque représenté schématiquement sur les figures 1 et 2 et désigné par la référence générale 10, comporte classiquement un boîtier 12 comprenant une source lumineuse 14 destinée à transmettre un rayonnement infrarouge à des tissus humains, et quatre photo-détecteurs 16a, 16b, 16c et 16d, tels que des photodiodes, disposés autour de la source lumineuse 14 de manière à recevoir le rayonnement infrarouge après son interaction avec les tissus. Le dispositif 10 comporte également un système de détection du mouvement 18, tel qu'un accéléromètre à trois dimensions, et un processeur 20 agencé pour recevoir et traiter les signaux issus des photo-détecteurs 16a, 16b, 16c, 16d, et de l'accéléromètre 18. Un module d'affichage 22, tel qu'un écran LCD, intégré au boîtier 12, est destiné à l'affichage des paramètres physiologiques mesurés, en particulier du rythme cardiaque. Le boîtier 12 est, en outre, muni d'un bracelet 24 pour la fixation du dispositif 10 au poignet à la façon d'une montre.

Selon l'invention, le dispositif 10 comporte encore, un sélecteur manuel de l'activité sportive pratiquée. Celui-ci se présente sous la forme d'un bouton poussoir 26 disposé sur un flanc du boîtier 12, permettant, par pressions successives, de sélectionner une activité parmi un choix d'activités présélectionnées. L'activité ainsi sélectionnée apparaît sur l'écran LCD sous la forme, par exemple, d'une icône.

Dans un mode de réalisation décrit mais non revendiqué, le dispositif 10 est exempt de sélecteur manuel de l'activité sportive, mais est agencé pour effectuer une sélection automatique à partir des signaux d'accélération. En effet, les signaux d'accélération contiennent une indication de l'activité pratiquée. L'analyse séparée en fréquence, en énergie et en forme, des signaux d'accélération dans les trois directions, permet de déterminer le type d'activité pratiquée. Comme précédemment, l'activité ainsi déterminée apparaît sur l'écran LCD sous la forme d'une icône.

Le dispositif 10 est destiné à la mesure d'un rythme cardiaque d'un sportif pratiquant un sport de préférence rythmique, tel que la marche, la course à pied, le cyclisme, la natation etc, ou tout autre sport associé à un mouvement périodique. Son fonctionnement est le suivant : L'utilisateur entre tout d'abord une indication sur l'activité sportive pratiquée, à l'aide du bouton poussoir 26. La source lumineuse 14 émet un rayonnement infrarouge qui interagit avec les tissus humains. Les photo-détecteurs 16a, 16b, 16c et 16d captent un signal optique contenant une information sur les tissus traversés. Parallèlement, l'accéléromètre 18 capte un signal d'accélération contenant une information sur le mouvement sportif effectué. Les signaux optique et d'accélération sont traités par le processeur 20, en vue d'extraire une information utile sur un paramètre physiologique, en particulier un rythme cardiaque. Le traitement des signaux optique et d'accélération est adapté en fonction de l'indication sur l'activité sportive pratiquée fournie par l'utilisateur. Dans la variante de ce mode de réalisation décrite mais non revendiquée, le signal d'accélération est tout d'abord traité, en vue de déterminer automatiquement le type d'activité pratiquée.

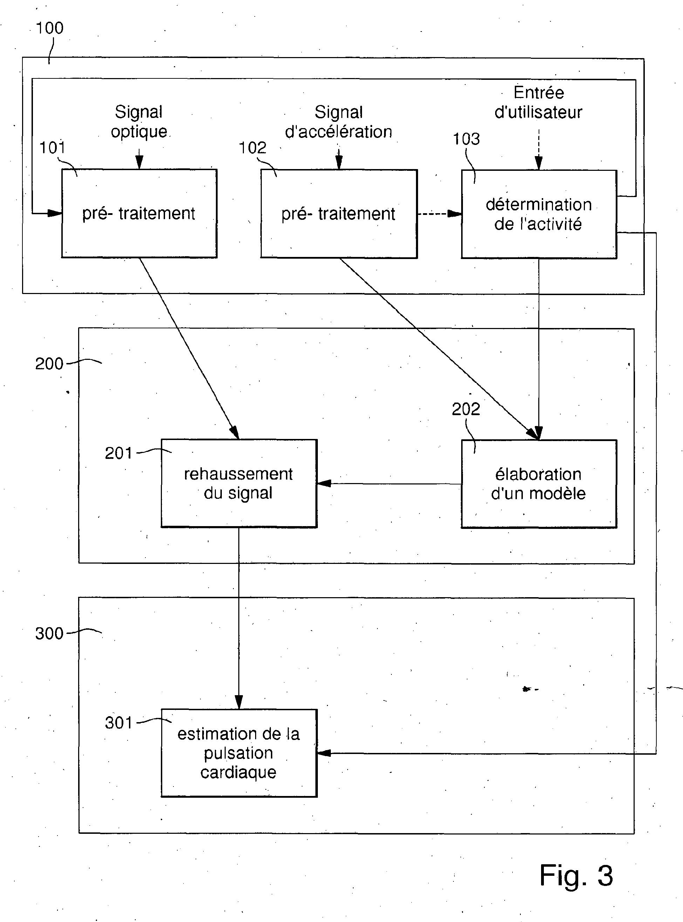

Quel que soit le mode de détermination de l'activité sportive pratiquée, le processeur 20 met en oeuvre un algorithme de traitement du signal illustré schématiquement en figure 3. Celui-ci comprend trois blocs de traitement principaux, respectivement de prétraitement 100, de rehaussement du signal optique 200, et d'estimation de la pulsation cardiaque 300, comportant chacun un certain nombre de sous-blocs, et détaillés par la suite. Cet algorithme s'applique à des méthodes de rehaussement du signal variées, par exemple, les méthodes citées précédemment. Néanmoins, dans un souci de simplification, il sera détaillé dans le cadre d'une méthode de calcul originale, décrite, par ailleurs, dans la demande EP06121865 déposée conjointement. On notera que ladite méthode est adaptée à la pratique d'un sport de type rythmique.

### a. Prétraitement 100

Les signaux optique et d'accélération issus respectivement des photo-détecteurs 16a, 16b, 16c, 16d, et de l'accéléromètre 18, subissent en premier lieu un prétraitement, symbolisé respectivement par deux sous-blocs 101 et 102 appartenant au bloc 100. Le prétraitement 101 consiste généralement, pour le signal optique, en une première étape d'élimination de la composante continue du signal, suivie d'une deuxième étape de filtrage en vue de réduire le domaine de fréquences aux valeurs compatibles avec un rythme cardiaque. Concernant le signal d'accélération, on procède essentiellement lors de l'étape de prétraitement 102 à la suppression de la composante continue. On notera que le prétraitement des signaux optique et d'accélération peut être effectué en mode analogique ou numérique.

Parallèlement, l'activité sportive pratiquée est déterminée lors d'une étape symbolisée par un sous-bloc 103, soit sur la base de la sélection effectuée par l'utilisateur, soit automatiquement, à partir des signaux d'accélération.

Un signal représentatif de l'activité pratiquée est transmis du sous-bloc 103 au sous-bloc 101 où il est utilisé pour le prétraitement du signal optique. Le filtrage est ainsi adapté en tenant compte du fait que, pour une activité donnée, le rythme cardiaque se situe à l'intérieur d'une plage donnée. A titre d'exemple, la bande passante pour la course à pied, se situe de préférence entre 1 Hz et 3.5 Hz, alors qu'elle est comprise entre 1 Hz et 3 Hz pour la marche.

### b. Rehaussement du signal optique 200

Le bloc de rehaussement du signal optique 200 comporte un sous-bloc 201 de rehaussement du signal proprement dit, et un sous-bloc 202 d'élaboration d'un modèle. A l'intérieur de ces deux sous-blocs, le traitement du signal est effectué, de préférence, en mode digital.

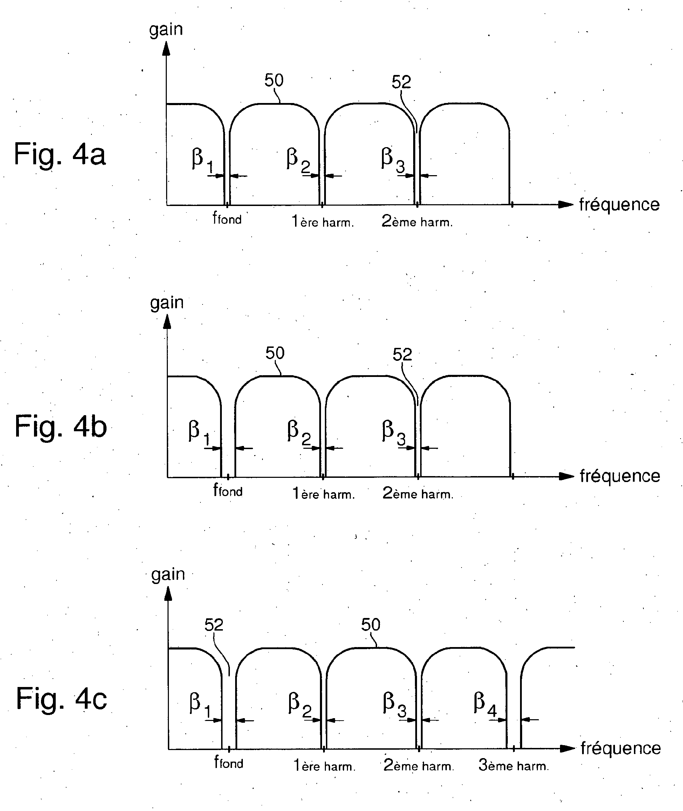

Une fois l'étape de prétraitement effectuée en 102, le signal d'accélération parvient au bloc 202 d'élaboration d'un modèle. Dans le cadre de la méthode originale décrite dans la demande EP06121865, l'élaboration d'un modèle à partir du signal d'accélération comprend une première étape de calcul de la fréquence fondamentale du mouvement, une deuxième étape de calcul des harmoniques à partir de la fréquence fondamentale, et une troisième étape d'élimination des fréquences ainsi calculées, du signal optique. Le fondement de cette méthode, ainsi que les détails du calcul sont explicités dans la demande EP06121865 mentionnée précédemment. On se bornera à mentionner que le calcul de la fréquence fondamentale est effectué par une méthode d'auto-corrélation, bien connue de l'homme de métier, suivie d'une détection des maxima. Les harmoniques sont ensuite calculées par une simple multiplication. Enfin, les fréquences du mouvement ainsi déterminées sont utilisées pour la construction d'un filtre en peigne tel que représenté en figure 4a. On reviendra ultérieurement sur les caractéristiques dudit filtre en peigne.

Les signaux issus des sous-blocs respectivement de prétraitement du signal optique 101 et d'élaboration d'un modèle 202, parviennent au sous-bloc 201 de rehaussement du signal optique. L'opération de rehaussement consiste, dans le cadre de la méthode décrite dans la demande EP06121865, en un filtrage du signal optique prétraité à l'aide du filtre en peigne construit lors de l'étape d'élaboration d'un modèle 202.

Selon l'invention, le filtre en peigne est construit à partir des informations sur les fréquences du mouvement, et de l'information relative au type d'activité pratiquée, délivrée par le sous-bloc 103. Il est formé classiquement de larges bandes passantes 50 séparées par d'étroites bandes de réjection 52 situées autour des fréquences du mouvement constituées par la fréquence fondamentale et les harmoniques. La largeur de chaque bande de réjection 52 est réglée par un paramètre βi, qui est adapté en fonction du niveau de l'harmonique et du type d'activité pratiquée.

A titre d'exemple, la figure 4b illustre un filtre peigne adapté à la course à pied. On note que le paramètre β1, lié à la fréquence fondamentale de la course à pied, génère une bande de réjection de la fréquence fondamentale relativement large, en comparaison des bandes de réjection des harmoniques, et en particulier de la première harmonique. Ceci s'explique par le fait que la fréquence fondamentale de la course à pied provient essentiellement du mouvement des bras. Or, ce mouvement est, d'une manière générale, moins régulier que le mouvement des jambes, qui est, lui, responsable en grande partie de la première harmonique. Par conséquent, le mouvement des bras génère un artefact plus large en fréquence que le mouvement des jambes. Il est donc nécessaire d'éliminer une plus grande portion de signal autour de la fréquence fondamentale, qu'autour de la première harmonique. Cette élimination de bruit se fait sans perte d'information utile, car la fréquence fondamentale du mouvement de la course est proche de 1 Hz, valeur éloignée de la plage des fréquences cardiaques habituelles pour une telle activité.

Un deuxième exemple illustré par la figure 4c, concerne un filtre en peigne adapté au cyclisme. Les paramètres β2 et β3 liés respectivement à la première et à la deuxième harmonique du cyclisme, génèrent deux bandes de réjection étroites, en comparaison des bandes de réjection des autres fréquences. Ce filtre a été construit essentiellement sur la base de données statistiques sur le mouvement du cycliste.

La construction d'un filtre adapté à l'activité sportive pratiquée, permet ainsi d'optimiser le rehaussement du signal optique.

### -c. Estimation de la pulsation cardiaque 300

Le signal optique rehaussé est filtré, à l'aide d'un filtre passe-bande adaptatif, ou d'un algorithme de réduction du bruit de type PCA tel que décrit dans le brevet US 7 018 338. Puis, la pulsation cardiaque est estimée à partir du signal optique rehaussé filtré. Pour cela on utilise, par exemple, par la méthode du 'zero-crossing' en terminologie anglaise, c'est-à-dire la méthode du passage à zéro, bien connue de l'homme de métier.

La pulsation cardiaque ainsi estimée est moyennée sur une fenêtre de temps dont la largeur dépend de l'activité pratiquée. A cet effet, un signal issu du bloc 103 de détermination de l'activité pratiquée est transmis à un bloc 301 d'estimation de la pulsation cardiaque. La largeur de la fenêtre de moyenne est ainsi sensiblement plus courte pour une activité telle que la course à pied, compatible avec des variations rapides du rythme cardiaque, que pour une activité telle que la marche, pour laquelle le rythme cardiaque varie lentement.

Dans une variante de ce mode de réalisation, la pulsation cardiaque estimée fait l'objet d'une auto-régression, et la constante de régression α est adaptée en fonction de l'activité pratiquée.

On a ainsi décrit un dispositif et une méthode de mesure d'une pulsation cardiaque dont les performances sont augmentées grâce à la détermination du type d'activité sportive pratiquée. Bien que le dispositif et la méthode décrits fassent intervenir une méthode de rehaussement du signal optique particulière, on notera que toute autre méthode de rehaussement du signal optique est compatible avec le dispositif et la méthode décrits. On a mentionné, par exemple, une méthode de rehaussement du signal optique par transformation de Fourier, décrite dans le document WO 2006/044677. Cette méthode consiste à calculer les spectres en fréquence des signaux optique et d'accélération, puis à éliminer du signal optique, les fréquences appartenant au signal d'accélération et mises en évidence par le calcul de la transformée de Fourier. Seules les fréquences pour lesquelles l'intensité du signal dépasse une certaine valeur seuil sont coupées dans le signal optique. Dans le cadre de l'invention, ladite valeur seuil dépend de l'activité pratiquée.

Bien entendu, la méthode et le dispositif selon l'invention ne se limitent pas au mode de réalisation qui vient d'être décrit et diverses modifications et variantes simples peuvent être envisagées par l'homme du métier sans sortir du cadre de l'invention tel que défini par les revendications annexées.

On notera, par exemple, que l'algorithme peut prendre en compte, non seulement l'activité pratiquée à l'instant présent, mais également son historique. En effet, il a été observé, dans le cadre de cette invention, que le mouvement sportif est moins régulier dans les premières minutes d'activité, que par la suite. On tiendra compte de ce paramètre, en construisant un filtre en peigne dont les bandes de réjection sont plus larges en début d'activité, qu'après quelques minutes. De plus, lors d'un changement d'activité, par exemple lors du passage de la marche à la course, on peut anticiper des variations rapides du rythme cardiaque. Dans les minutes qui suivent un tel changement, la fenêtre de moyenne ou la constante de régression seront adaptées pour tenir compte d'une possible variation brutale du rythme cardiaque.

## Revendications

1. Méthode de mesure d'une pulsation cardiaque durant une activité sportive, consistant principalement à :
- transmettre un rayonnement lumineux dans des tissus organiques,
- détecter un signal optique issu de l'interaction dudit rayonnement lumineux avec les tissus organiques, ledit signal optique comprenant une composante relative à la pulsation cardiaque et une composante relative au mouvement effectué durant ladite activité sportive,
- détecter un signal d'accélération contenant une indication relative au mouvement effectué durant ladite activité sportive,
- sélectionner le type d'activité sportive pratiquée parmi un choix d'activités préselectionnées à l'aide d'un sélecteur manuel et
- calculer ladite pulsation cardiaque à partir dudit signal optique et à l'aide dudit signal d'accélération, par séparation des composantes relatives, respectivement, à la pulsation cardiaque et au mouvement effectué, ledit calculde la pulsation cardiaque étant adapté en fonction du type d'activité pratiquée, ledit calcul de la pulsation cardiaque étant basé sur un algorithme comprenant un bloc de traitement pour le prétraitement des signaux optique et d'accélération (100), ledit bloc de prétraitement (100) comprenant une étape de filtrage du signal optique à l'aide d'un filtre dont la largeur est adaptée au type d'activité pratiquée, en tenant compte du fait que pour une activité donnée, le rythme cardiaque se situe à l'intérieur d'une plage donnée,
ledit calcul de la pulsation cardiaque étant basé sur un algorithme comprenant un bloc de traitement pour séparer des composantes relatives, respectivement, à la pulsation cardiaque et au mouvement effectué, appelé bloc de rehaussement du signal optique (200), ledit bloc de rehaussement du signal optique (200) comprenant:
- une étape de calcul de la fréquence fondamentale du mouvement,
- une étape de calcul des harmoniques, et
- une étape d'élimination des fréquences du mouvement ainsi calculées par filtrage dudit signal optique à l'aide d'un filtre de type peigne,
ledit filtre en peigne comprenant des bandes passantes (50) et des bandes de réjection (52), la largeur desdites bandes (50, 52) étant adaptée au type d'activité pratiquée.

2. Méthode selon la revendication 1, **caractérisée en ce que** ledit filtre en peigne est adapté à la course à pied et comprend une première bande de réjection large en comparaison de la bande de réjection de la première harmonique.

3. Méthode selon la revendication 2, **caractérisée en ce que** ledit filtre adapté à la course à pied est passant pour les fréquences comprises entre 1 Hz et 3.5 Hz.

4. Méthode selon l'une des revendications 1 à 3, **caractérisée en ce que** ledit calcul de la pulsation cardiaque est basé sur un algorithme comprenant un bloc de traitement pour l'estimation de la pulsation cardiaque (300), ledit bloc d'estimation de la pulsation cardiaque (300) comprenant au moins une étape adaptée au type d'activité pratiquée.

5. Méthode selon la revendication 4, **caractérisée en ce que** l'estimation de la pulsation cardiaque est moyennée sur une fenêtre de temps dont la largeur est adaptée en fonction de l'activité pratiquée.

6. Méthode selon l'une des revendications 1 à 5, **caractérisée en ce que** ledit calcul de la pulsation cardiaque est adapté en fonction de l'historique de l'activité pratiquée.

7. Méthode selon les revendications 1 et 4, **caractérisée en ce que** lesdites bandes de réjection (52) sont plus larges en début d'activité que par la suite.

8. Dispositif intégré pour la mise en oeuvre de la méthode selon les revendications 1 à 7, comportant :
- une source lumineuse,
- un photo-détecteur fournissant un signal optique comprenant une composante relative à ladite pulsation cardiaque et une composante relative au mouvement effectué durant ladite activité sportive,
- un accéléromètre fournissant un signal d'accélération contenant une indication relative au mouvement effectué durant ladite activité sportive, et
- un processeur apte à traiter lesdits signaux de manière à calculer ladite pulsation cardiaque à partir dudit signal optique et à l'aide du signal d'accélération, par séparation des composantes relatives, respectivement, à la pulsation cardiaque et au mouvement effectué, des moyens de détermination de ladite activité sportive comportant un sélecteur manuel de l'activité sportive pratiquée formé par un organe de commande externe, lesdits moyens de détermination de ladite activité sportive étant agencés pour fournir au processeur une indication du type d'activité pratiquée pour adapter le calcul de la pulsation cardiaque en fonction de l'indication du type d'activité.

## Patentansprüche

1. Verfahren zum Messen eines Herzpulses während einer sportlichen Aktivität, das hauptsächlich darin besteht:
- Lichtstrahlung in organische Gewebe zu senden,
- ein aus der Wechselwirkung der Lichtstrahlung mit den organischen Geweben stammendes optisches Signal zu detektieren, wobei das optische Signal eine auf den Herzpuls bezogene Komponente und eine Komponente, die auf die während der sportlichen Aktivität ausgeführte Bewegung bezogen ist, umfasst,
- ein Beschleunigungssignal zu detektieren, das eine Angabe bezüglich der während der sportlichen Aktivität ausgeführten Bewegung enthält,
- den Typ der praktizierten sportlichen Aktivität aus einer Auswahl im Voraus ausgewählter Aktivitäten mit Hilfe eines manuellen Selektors auszuwählen und
- den Herzpuls anhand des optischen Signals und mit Hilfe des Beschleunigungssignals durch Trennen von Komponenten, die auf den Herzpuls bzw. die ausgeführte Bewegung bezogen sind, zu berechnen, wobei das Berechnen des Herzpulses als Funktion des Typs der praktizierten Aktivität angepasst wird, wobei das Berechnen des Herzpulses auf einem Algorithmus beruht, der einen Verarbeitungsblock (100) für die Vorverarbeitung des optischen Signals und des Beschleunigungssignals enthält, wobei der Vorverarbeitungsblock (100) einen Schritt des Filterns des optischen Signals mit Hilfe eines Filters dessen Breite an den Typ der praktizierten Aktivität angepasst ist, unter Berücksichtigung der Tatsache, dass der Herzrhythmus für eine gegebene Aktivität innerhalb eines gegebenen Bereichs liegt, umfasst,
wobei die Berechnung des Herzpulses auf einem Algorithmus beruht, der einen Verarbeitungsblock enthält, um die Komponenten, die auf den Herzpuls bzw. auf die ausgeführte Bewegung bezogen sind zu trennen, der Block (200) zum Verstärken des optischen Signals genannt wird, wobei der Block (200) zum Verstärken des optischen Signals Folgendes umfasst:
- einen Schritt des Berechnens der Grundfrequenz der Bewegung,
- einen Schritt des Berechnens der Harmonischen und
- einen Schritt des Beseitigens der so berechneten Frequenzen der Bewegung durch Filtern des optischen Signals mit Hilfe eines Kammfilters,
wobei das Kammfilter Durchlassbänder (50) und Sperrbänder (52) enthält, wobei die Breite der Bänder (50, 52) an den Typ der praktizierten Aktivität angepasst ist.

2. Verfahren nach Anspruch 1, **dadurch gekennzeichnet, dass** das Kammfilter an das Laufen angepasst ist und ein erstes Sperrband aufweist, das im Vergleich zu dem Sperrband der ersten Harmonischen breit ist.

3. Verfahren nach Anspruch 2, **dadurch gekennzeichnet, dass** das an das Laufen angepasste Filter Frequenzen, die im Bereich von 1 Hz bis 3,5 Hz liegen, durchlässt.

4. Verfahren nach einem der Ansprüche 1 bis 3, **dadurch gekennzeichnet, dass** die Berechnung des Herzpulses auf einem Algorithmus beruht, der einen Verarbeitungsblock (300) zum Schätzen des Herzpulses enthält, wobei der Block (300) zum Schätzen des Herzpulses wenigstens einen Schritt umfasst, der an den Typ der praktizierten Aktivität angepasst ist.

5. Verfahren nach Anspruch 4, **dadurch gekennzeichnet, dass** das Schätzen des Herzpulses in einem Zeitfenster, dessen Breite als Funktion der praktizierten Aktivität angepasst wird, gemittelt wird.

6. Verfahren nach einem der Ansprüche 1 bis 5, **dadurch gekennzeichnet, dass** das Berechnen des Herzpulses als Funktion der Historie der praktizierten Aktivität angepasst wird.

7. Verfahren nach den Ansprüchen 1 und 4, **dadurch gekennzeichnet, dass** die Sperrbänder (52) zu Beginn der Aktivität breiter als in der Folge sind.

8. Integrierte Vorrichtung zum Ausführen des Verfahrens nach den Ansprüchen 1 bis 7, die umfasst:
- eine Lichtquelle,
- einen Photodetektor, der ein optisches Signal liefert, das eine Komponente, die auf den Herzpuls bezogen ist, und eine Komponente, die auf die während der sportlichen Aktivität ausgeführte Bewegung bezogen ist, enthält,
- einen Beschleunigungsmesser, der ein Beschleunigungssignal liefert, das eine Angabe bezüglich der während der sportlichen Aktivität ausgeführten Bewegung enthält, und
- einen Prozessor, der die Signale in der Weise verarbeiten kann, dass der Herzpuls anhand des optischen Signals und mit Hilfe des Beschleunigungssignals durch Trennen der Komponenten, die auf den Herzpuls bzw. auf die ausgeführte Bewegung bezogen sind, berechnet wird,
- Mittel zum Bestimmen der sportlichen Aktivität einen manuellen Selektor für die praktizierte sportliche Aktivität enthalten, der durch ein externes Steuerorgan gebildet ist, wobei die Mittel zum Bestimmen der sportlichen Aktivität dafür ausgelegt sind, dem Prozessor eine Angabe der praktizierten Aktivität zu liefern, um die Berechnung des Herzpulses als Funktion der Angabe des Aktivitätstyps anzupassen.

## Claims

1. Method of measuring a cardiac pulsation during a sporting activity, mainly comprising the steps of:
- transmitting light radiation into organic tissue,
- detecting an optical signal derived from the interaction of said light radiation with the organic tissue, said optical signal including one component relating to the cardiac pulsation and one component relating to said movement carried out during said sporting activity,
- detecting an acceleration signal containing an indication relating to said movement carried out during said sporting activity,
- selecting the type of sporting activity being practised among a set of preselected activities, by means of a manual selector, and
- calculating said cardiac pulsation from said optical signal and using said acceleration signal, by separating the components respectively relating to the cardiac pulsation and to the movement carried out, said cardiac pulsation calculation being adapted as a function of the type of sporting activity being practised, said calculation of the cardiac pulsation being based on an algorithm including a processing block for the pre-processing of the optical and acceleration signals (100), said pre-processing block (100) comprising a step of filtering the optical signal using a filter whose width is adapted to the type of activity being practiced, the fact that for a given activity the heart rate is in a given range, being taken into account,
said calculation of the cardiac pulsation being based on an algorithm including a processing block to separate components respectively relating to the cardiac pulsation and to the movement carried out, called the optical signal enhancement block (200), said optical signal enhancement block (200) including:
- a step of calculating the fundamental frequency of the movement,
- a step of calculating the harmonics, and
- a step of removing the movement frequencies thereby calculated by filtering of said optical signal using a comb type filter,
said comb filter including pass-bands (50) and rejection bands (52), the width of said bands (50, 52) being adapted to the type of activity being practised.

2. Method according to claim 1, wherein said comb filter is adapted to running and includes a first wide rejection band in comparison to the rejection band of the first harmonic.

3. Method according to claim 2, wherein said filter adapted to running is passing for frequencies comprised between 1 Hz and 3.5 Hz.

4. The method according to any of claims 1 to 3, wherein the cardiac pulsation calculation is based on an algorithm including a processing block for estimating the cardiac pulsation (300), said cardiac pulsation estimation block (300) including at least one step adapted to the type of activity being practised.

5. Method according to claim 4, wherein the cardiac pulsation estimation is averaged over a time window whose width is adapted as a function of the activity being practised.

6. Method according to any of claims 1 to 5, wherein said cardiac pulsation calculation is adapted as a function of the history of the activity being practised.

7. Method according to any of claims 1 to 4, wherein said rejection bands (52) are wider at the start of activity than thereafter.

8. Integrated device for implementing the method according to claims 1 to 7, including:
- a light source,
- a photo sensor supplying an optical signal including one component relating to said cardiac pulsation and one component relating to the movement carried out during said sporting activity,
- an accelerometer supplying an acceleration signal containing an indication relating to the movement carried out during said sporting activity, and
- a processor able to process said signals so as to calculate said cardiac pulsation from said optical signal and using the acceleration signal, by separating the components respectively relating to the cardiac pulsation and to the movement carried out,
- means for determining said sporting activity comprising a manual selector of the activity being practised formed by an external control member, said means for determining said sporting activity being arranged to provide the processor with an indication of the type of the activity being practised so as to adapt the cardiac pulsation calculation as a function of the indication of the type of activity.
